# EUROPÄISCHE PATENTSCHRIFT

(11) **EP 3 863 702 B1**
(45) Veröffentlichungstag und Bekanntmachung des Hinweises auf die Patenterteilung: **09.08.2023**
(21) Anmeldenummer: 19794408.5
(22) Anmeldetag: 03.10.2019
(51) Int. Cl.: A61M 11/04, A61M 16/10, A61M 16/16, A61M 13/00

(54) **WASSERRESERVOIR FÜR EINE VORRICHTUNG ZUR GASBEFEUCHTUNG IN DER LAPAROSKOPIE**
WATER RESERVOIR FOR A DEVICE FOR GAS HUMIDIFICATION IN LAPAROSCOPY
RÉSERVOIR D'EAU POUR UN DISPOSITIF D'HUMIDIFICATION DE GAZ EN LAPAROSCOPIE

(30) Priorität: 10.10.2018 DE 102018007981
(43) Veröffentlichungstag der Anmeldung: 18.08.2021
(73) Patentinhaber: W.O.M. World of Medicine GmbH, 10587 Berlin (DE)
(72) Erfinder: SCHWARZ, Daniel, 13055 Berlin (DE)
(74) Vertreter: Jungblut & Seuss
(86) Internationale Anmeldenummer: PCT/DE2019/000257
(87) Internationale Veröffentlichungsnummer: WO 2020/074027

(56) Entgegenhaltungen:
- EP-A1- 3 363 489
- WO-A1-98/26826
- WO-A1-2009/015410
- WO-A1-2012/100291
- DE-A1- 19 510 710
- US-A- 6 068 609
- US-A1- 2003 181 857
- US-A1- 2006 012 057

## Beschreibung

Die vorliegende Erfindung betrifft ein Wasserreservoir für eine Vorrichtung zur Gasbefeuchtung in der Laparoskopie.

### Stand der Technik

Die Laparoskopie ist ein medizinischer Eingriff bei dem die Bauchhöhle und die darin liegenden Organe visuell überprüft werden können. Hierzu werden üblicherweise kleine Hautschnitte (0,3 - 2 cm) in die Bauchdecke gemacht und durch diese ein Trokar eingebracht, welcher wiederum eine optische Vorrichtung aufnehmen kann. Mit Hilfe eines speziellen Endoskops (Laparoskop) kann der Bauchraum eingesehen werden. Bei der diagnostischen Laparoskopie wird der Bauchraum lediglich visuell inspiziert, im Rahmen eines therapeutischen Vorgriffs können auch operative Eingriffe vorgenommen werden.

Üblicherweise wird zu Beginn der Laparoskopie zunächst der Bauchraum mit Gas befüllt, um ein Pneumoperitoneum zu schaffen. Hierzu sind bereits verschiedene Gase verwendet worden, wie zum Beispiel Luft, Stickstoff oder Kohlendioxid (CO₂). Die Verwendung von Kohlendioxidgas hat sich besonders gut bewährt. Es wurde festgestellt, dass es, insbesondere bei längeren laparoskopischen Eingriffen, sinnvoll ist das eingeführte Gas einerseits zu erwärmen und andererseits zu befeuchten. Die Gaserwärmung dient dazu, den Patienten nicht abzukühlen, sowie ein diffuses Schmerzgefühl des Patienten zu vermeiden, welches wahrscheinlich eine Folge lokaler Abkühlung in Folge des Eintritts von kaltem Gas ist. Die Befeuchtung dient dazu, einem Austrocknen der inneren Bauchoberflächen vorzubeugen, auch um die dabei entstehende Abkühlung zu vermeiden.

Hierzu sind im Stand der Technik bereits Anregungen gegeben. In der US 6,068,609 ist beispielsweise eine Vorrichtung beschrieben, die eine Gaserwärmung und -befeuchtung für die Laparoskopie ermöglicht. Hierin wird eine separate Kammer beschrieben, die mit einer Widerstandheizung ausgestattet ist. In der Kammer befindet sich zusätzlich ein absorbierendes Material, wie zum Beispiel ein Schwamm, der befeuchtet werden kann.

Eine andere Ausführungsform einer Vorrichtung für die Gasbefeuchtung im Rahmen der Laparoskopie wird beispielsweise in der US 2003/0181857 A1 vorgestellt. Hier befindet sich im Inneren eines Gasschlauches ein saugfähiges Material (wick). Wenn das Gas aus dem Insufflator durch den Schlauch strömt, wird es gleichzeitig befeuchtet. Voraussetzung hierfür ist, dass das im Schlauch befindliche Material während der Operation gleichmäßig feucht gehalten wird. Für diesen Zweck sieht das Dokument einen Vorratsbehälter mit Wasser vor, welches oberhalb des Schlauches angeordnet ist. Über einen Schlauch soll dann Wasser auf das Befeuchtungsmaterial abgegeben werden.

Weiterer Stand der Technik wird in den Druckschriften US 2013/0239966 A1, EP 0934091 A1 und DE 19510710 A1 beschrieben.

In der Praxis hat sich herausgestellt, dass die gleichmäßige Versorgung mit Feuchtigkeit ein elementares Problem dieser Art von Gasbefeuchtungsvorrichtung ist. Je nach Art der Operation und dem hierfür notwendigen Gasfluss wird aus dem Wasserbehälter häufig entweder zu viel oder zu wenig Wasser abgegeben

Es stellt sich daher das Problem, für eine im Rahmen der Laparoskopie verwendete Insufflationsvorrichtung bestehend aus einer Gasversorgungseinrichtung und einem Gasschlauch, der Gas zum Patienten liefert, wobei der Schlauch optional ein Befeuchtungsmaterial enthalten kann, ein Wasserreservoir anzugeben, welches in der Lage ist, die enthaltene Wassermenge gleichmäßig an das strömende Gas abzugeben.

Weiterhin soll die Gerätehandhabung für das medizinische Personal nicht erschwert werden. Aus diesem Grunde soll die Erfindung es ermöglichen, dass das Wasserreservoir während laufender Insufflation nachgefüllt werden kann. Das Wasserreservoir kann entweder nahe am Gerät oder mit einem Abstand zum Patienten am Schlauchset angebracht werden. Der Vorteil der gerätenahen Positionierung liegt darin, dass das Wasserreservoir bei der Behandlung weniger stört, der Vorteil der patientennahen Positionierung liegt darin, dass weniger Kondensation im Schlauch auftritt.

Aufgabe der vorliegenden Erfindung ist es daher, eine einfachere Vorrichtung zur Gaserwärmung und -befeuchtung zur Verfügung zu stellen, die die oben genannten Nachteile vermeidet.

### Lösung der Aufgabe

Die Lösung dieser Aufgabe erfolgt durch den Gegenstand des Patentanspruchs 1, nämlich
eine Vorrichtung zur Gasbefeuchtung in der Laparoskopie zum Anschluss an eine Gasversorgungsvorrichtung (Insufflator), enthaltend
ein Wasserreservoir (1) in einer Kunststoffkammer (2) mit optionalem ersten saugfähigem Befeuchtungsmittel (3),
wobei die Kunststoffkammer einen Gaseingang (4) und einen Gasausgang (5) aufweist,
wobei der Gaseingang direkt oder über einen optionalen Schlauch (6) mit dem Insufflator verbunden ist und wobei der Gasausgang zu einem Gasschlauch (7) führt,
ein oder mehrere im wesentlichen zylinderförmige Befeuchtungsmittel aus einem zweiten saugfähigen Material (8),
einen Gasschlauch (7), über den Gas in einen Patienten geführt werden kann, dadurch gekennzeichnet, dass die ein oder mehreren im wesentlichen zylinderförmigen Befeuchtungsmittel (8) aus einem zweiten saugfähigen Material mit mindestens einer Zylinderstirnfläche (9) in Kontakt mit dem Wasserreservoir (1) stehen und wobei die andere Zylinderstirnfläche oder der Zylindermantel (10) im Gasstrom positioniert sind und wobei das Wasserreservoir mit dem ersten Befeuchtungsmittel (3) konzentrisch um den Gasstrom herum angeordnet ist.

Bevorzugt steht mindestens eines der Befeuchtungsmittel in Verbindung mit einem Heizelement oder ist umwickelt durch einen Heizdraht (11). Die Befeuchtungskammer verfügt über einen optionalen Nachfüllanschluss (12). Vorteilhafte Ausgestaltungen sind Gegenstand der Unteransprüche.

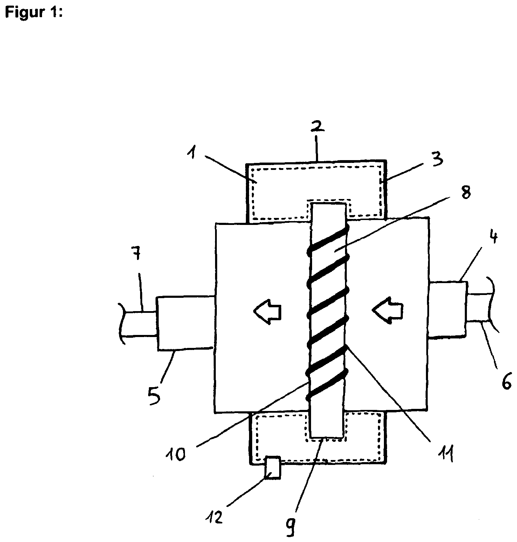

Kern der vorliegenden Erfindung ist eine wasserenthaltende Kammer, die zwischen der Gasversorgungseinrichtung (Insufflator) und dem zum Patienten führenden Schlauch angeordnet ist. Diese Kammer kann Wasser in flüssiger Form enthalten. Bevorzugt ist das Wasser in einem Befeuchtungsmaterial (z.B. Baumwollwatte, Schwamm, etc.) gespeichert. Aus der Kammer wird das Wasser durch die Kapillarwirkung eines zweiten Befeuchtungsmittels (z.B. Baumwolltamponade) in den Gasstrom geleitet (siehe Figur 1). Es hat sich herausgestellt, dass der Feuchtigkeitstransport durch die Kapillarwirkung besonders gleichmäßig erfolgt. Verdunstendes Wasser an der Oberfläche des Befeuchtungsmaterials wird in Abhängigkeit vom Verbrauch gleichmäßig nachgeliefert. Im Ergebnis wird eine besonders gleichmäßige Befeuchtung des Gasstroms erzielt. Bevorzugt ist das zweite Befeuchtungsmittel im Wesentlichen zylinderförmig geformt. Es kann aber auch eine von der Zylinderform abweichende Form aufweisen, z.B. als sechseckige Säule ausgestaltet sein. Die Enden der Säule bzw. des Zylinders können auch abgerundet sein.

Je nach durchzuleitender Gasmenge wird unterschiedlich viel Wasser verbraucht. Es hat sich herausgestellt, dass es bei Insufflatoren mit höheren Gasvolumenströmen (20 - 50 l/Min.) von Vorteil sein kann, mehrere Verbindungen zwischen Kammer und Heizeinheit einzusetzen, beispielsweise drei bis vier zylinderförmige Befeuchtungsmittel (z.B. Tamponaden mit einem Durchmesser von ein bis zwei Zentimeter und einer Länge von 4-6 cm). Bei Insufflatoren mit geringerem Gasvolumenstrom können ein bis zwei derartige Elemente ausreichend sein.

In vielen Fällen ist es ausreichend, wenn der Gasstrom über die Zylinderstirnfläche der Befeuchtungsmittel (z.B. Tamponade) geführt wird, um zu einer ausreichenden Befeuchtung zu kommen (in den Figuren nicht dargestellt). In anderen Fällen wird der Gasstrom direkt über die Zylindermantelfläche der zylinderförmigen Befeuchtungsmittel geführt, wie es in Figur 1 dargestellt ist. Bevorzugt wird die Tamponade direkt mit Heizdraht umwickelt und wirkt als zusätzliche Heizeinheit.

Optional kann das oder können die zylinderförmigen Befeuchtungsmittel geheizt sein, beispielsweise durch enthaltene oder umwickelnde Heizdrähte, die durch elektrische Widerstandsheizung beheizt werden. In Figur 1 dargestellt ist die spiralförmige Umwicklung einer Tamponade mit einem Heizdraht.

Optional kann der Gasschlauch ebenfalls ein Befeuchtungsmaterial enthalten (z. B. einen im Gasschlauch enthaltenen Viskosefliess-Streifen). In diesem Fall ist es zweckmäßig, wenn das Material in direktem Kontakt mit den zylinderförmigen Befeuchtungsmitteln steht. In diesem Fall kann das durch Kapillarwirkung aufsteigende Wasser über den Viskosefliess-Streifen weitergeleitet werden. Es ist möglich, gleichzeitig eine Heizvorrichtung im Gasschlauch anzubringen, die das durch den Schlauch geleitete Gas aufheizt. Die Heizung kann gleichzeitig den enthaltenen Viskosefliess-Streifen heizen und so Erwärmung und Befeuchtung des Gases sicherstellen.

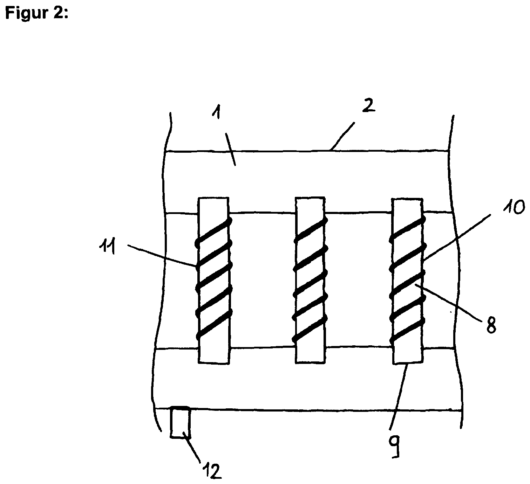

Erfindungsgemäß ist das Wasserreservoir auch konzentrisch um den Gasstrom herum angeordnet (Figuren 1-3). Hierzu kann beispielsweise ein absorbierendes Material, z.B. Baumwolle, in einer im Wesentlichen runden Kammer angeordnet sein, wobei der Gasstrom axial durch den Kern der Kammer geführt wird. Auch in diesem Fall kann die Feuchtigkeit durch das enthaltene Material der Kammer durch zylinderförmige Befeuchtungsmittel (z.B. Tamponaden) in den Gasstrom geleitet werden. In diesem Fall sind die Tamponaden beispielsweise orthogonal zum Gasstrom angeordnet, so dass der Gasstrom über die äußere Mantelfläche der zylinderförmigen Tamponade geführt wird. Auch in diesem Fall kann eine elektrische Widerstandsheizung vorgesehen sein, die beispielsweise durch eine spiralförmige Ummantelung der Tamponade mit einem Heizdraht eingerichtet ist. Auch diesem Fall sorgt die Kapillarwirkung der Tamponade durch eine besonders gleichmäßige Befeuchtung des Gasstroms. Vorteil dieser Ausführungsform ist, dass die Befeuchtungsleistung unabhängig von der Lage der Kammer ist. Es können auch mehrere Tamponaden nacheinander in den Gasstrom positioniert werden Hierbei können die Tamponaden parallel zueinander angeordnet sein (Figur 2).

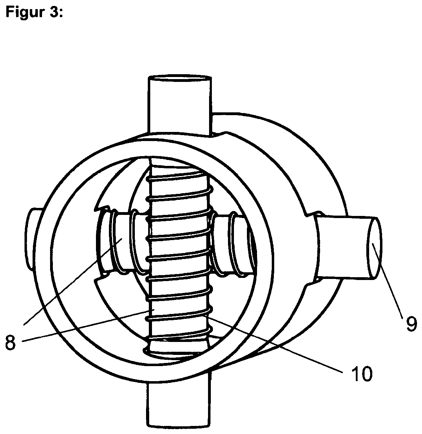

Bevorzugt sind sie jedoch nicht parallel angeordnet, sondern in einem Winkel α von α = 180° : n, wobei n die Anzahl der Tamponaden angibt: Bei zwei Tamponaden hat sich ein Winkel von 90° als optimal erwiesen (Figur 3), bei drei Tamponaden hat sich ein Winkel von jeweils 60° als optimal erwiesen.

In jedem Fall verfügt die Kammer über entsprechend angepasste Halteelemente für die Befeuchtungsmittel.

Selbstverständlich kann die derart gestaltete Kammer eine Nachfüllvorrichtung enthalten, beispielsweise gebildet durch einen Stutzen, der mittels einer Spritze befüllt werden kann.

Dem Fachmann ist natürlich unmittelbar klar, dass die Kammer dieser Befeuchtungsvorrichtung nicht exakt rund sein muss, auch andere Ausführungsformen sind möglich, beispielsweise quadratische, sechseckige oder achteckige Ausführungsformen.

Allen Ausführungsformen ist gemein, dass vor Beginn des laparoskopischen Eingriffs die ausreichende Befeuchtung aller Komponenten, d. h. des als Wasserreservoir dienenden Befeuchtungsmaterials und der Tamponade, sichergestellt werden muss. Das erfindungsgemäße Wasserreservoir wird üblicherweise in trockener Form gelagert und ausgeliefert. Vor Beginn der Operation muss daher eine Befüllung mit Flüssigkeit vorgenommen werden. Dies wird im Regelfall über den Einfüll- bzw. Nachfüllstutzen mit angeschlossenem Septum erfolgen. Alternativ kann nur das Wasserreservoir befeuchtet werden, wobei dann abzuwarten ist, bis die Tamponade komplett durchfeuchtet ist. Für den Fachmann auf dem Gebiet versteht sich von selbst, dass sowohl das vor Beginn der Operation, als auch ggf. während der Operation zugegebene Flüssigkeit steril sein muss.

Optional besteht die Möglichkeit, Wasserreservoir und/oder Tamponade mit einem Feuchtigkeitsindikator zu versehen, der beispielsweise durch eine Farbänderung anzeigt, wenn das Material befeuchtet ist. In diesem Fall wäre auf eine gleichmäßige Verfärbung zu achten, bevor die Operation beginnt.

Das erfindungsgemäße Wasserreservoir kann entweder unmittelbar am Gasausgang des Insufflator sitzen oder an dem Schlauch angebracht sein, der zum Patienten führt. Gängige Insufflatorenmodelle enthalten an diesem Gasausgang häufig einen separaten Filter. In diesem Fall kann das Reservoir unmittelbar an den Filter positioniert werden und ein durchgängiges Gehäuseteil für Filterhalterung und Reservoir bilden. Alternativ kann der Gasausgang des Insufflators durch einen Schlauch mit dem Gaseingang des Reservoirs verbunden werden.

Die Kammer für das Wasserreservoir kann aus den gängigen Materialien hergestellt werden, welche in der Medizintechnik verwendet werden (beispielsweise PVC, PE, PP, etc.), beispielsweise durch Spritzguss oder 3D-Druck. Die Kammer oder Teile hiervon können auch transparent gestaltet werden, um eine Sichtkontrolle zu ermöglichen.

Am Gasausgangsende des Wasserreservoirs ist der Heizschlauch positioniert. Dieser kann unmittelbar angeklebt oder angeschweißt sein. Eine andere Möglichkeit ist, dass die Gasausgangsseite der Kammer zu einem Stutzen ausgeformt ist, auf den der eigentliche Gasschlauch gesteckt und darauf fixiert wird.

Der Gasschlauch selbst wird in üblicher Weise aus Kunststoff gefertigt. Angesichts der zahlreichen Beschreibungen im Stand der Technik kann an dieser Stelle auf gesonderte Ausführungen verzichtet werden.

Selbstverständlich ist es möglich, den Gasschlauch mit einem Heizdraht zu versehen, der beispielsweise über die gesamte Länge des Heizschlauches eine gleichmäßige Beheizung des Gasstroms sicherstellt. Am distalen (patientenseitigen) Ende des Gasschlauches wird ein Anschluss für das medizinische Instrument vorgesehen, welches das Gas in den Körper leitet (z.B. Veress-Nadel oder Trokar).

Weiterhin optional kann der Gasschlauch mit einem Temperatursensor versehen sein, beispielsweise am patientenseitigen Ende des Gasschlauches um die resultierende Gastemperatur zu überwachen

### Beschreibung der Figuren

**Figur 1** zeigt den Querschnitt einer erfindungsgemäßen Gasbefeuchtungsvorrichtung mit einem im Wesentlichen zylinderförmigen Befeuchtungsmittel (8), welches von einem Heizdraht (11) umwickelt ist. Das erste Befeuchtungsmittel befindet sich ein einer runden Kunststoffkammer, die konzentrisch um den Gasstrom (Pfeile) angeordnet ist.
**Figur 2** zeigt eine Anordnungsmöglichkeit für mehrere (hier: drei) zylinderförmige Befeuchtungsmittel (8) im Gasstrom. Die zylinderförmigen Befeuchtungsmittel (8) sind in einer Weise parallel im Gasstrom angeordnet, dass der Gasstrom die Zylinder nacheinander passiert.
**Figur 3** zeigt eine andere Anordnungsmöglichkeit für mehrere (hier: zwei) zylinderförmige Befeuchtungsmittel (8) im Gasstrom. Die zylinderförmigen Befeuchtungsmittel (8) sind im Winkel von 90° zueinander angeordnet.

### Bezugszeichenliste

1) Wasserreservoir
2) Kunststoffkammer
3) erstes saugfähiges Befeuchtungsmittel
4) Gaseingang in die Kunststoffkammer
5) Gasausgang aus der Kunststoffkammer
6) optionaler Schlauch vom Insufflator zum Gaseingang (4) der Kunststoffkammer (2)
7) Schlauch vom Gasausgang (5) der Kunststoffkammer (2) Patienten
8) zweites saugfähiges Befeuchtungsmittel (im wesentlichen zylinderförmig)
9) Zylinderstirnfläche des im wesentlichen zylinderförmigen zweiten Befeuchtungsmittels (8)
10) Zylindermantelfläche des im wesentlichen zylinderförmigen zweiten Befeuchtungsmittels (8)
11)Heizdraht
12)Optionaler Zugang zur Kunststoffkammer (2) für die Befeuchtung des /der Befeuchtungsmittel

## Patentansprüche

1. (Hauptantrag vom 03.04.2023) 1,) Vorrichtung zur Gasbefeuchtung in der Laparoskopie zum Anschluss an eine Gasversorgungsvorrichtung (Insufflator), enthaltend
ein Wasserreservoir (1) in einer Kunststoffkammer (2) mit erstem saugfähigen Befeuchtungsmittel (3),
wobei die Kunststoffkammer einen Gaseingang (4) und einen Gasausgang (5) aufweist,
wobei der Gaseingang direkt oder über einen optionalen Schlauch (6) mit dem Insufflator verbunden ist und wobei der Gasausgang (5) zu einem Gasschlauch (7) führt,
ein oder mehrere im wesentlichen zylinderförmige Befeuchtungsmittel (8) aus einem zweiten saugfähigen Material,
einen Gasschlauch (7), über den Gas in einen Patienten geführt werden kann,
wobei die ein oder mehrere im wesentlichen zylinderförmigen Befeuchtungsmittel (8) aus einem zweiten saugfähigen Material mit mindestens einer Zylinderstirnfläche (9) in Kontakt mit dem Wasserreservoir (1) stehen, wobei die andere Zylinderstirnfläche oder der Zylindermantel (10) im Gasstrom positioniert sind und **dadurch gekennzeichnet, dass** das Wasserreservoir mit dem ersten Befeuchtungsmittel (3) konzentrisch um den Gasstrom herum angeordnet ist.

2. Vorrichtung gemäß Anspruch 1, **dadurch gekennzeichnet, dass** das erste und/oder das zweite Befeuchtungsmittel (3, 8) aus einem Material besteht, welches über Kapillarwirkung Flüssigkeit transportieren kann.

3. Vorrichtung gemäß Anspruch 1, **dadurch gekennzeichnet, dass** die Kunststoffkammer (2) einen Zugang (12) zum Befeuchten des oder der Befeuchtungsmittel (3, 8) enthält.

4. Vorrichtung gemäß mindestens einem der Ansprüche 1-3, **gekennzeichnet durch** einen beheizten Gasschlauch (7) am Gasausgang der Kunststoffkammer (2).

5. Vorrichtung gemäß mindestens einem der Ansprüche 1-4, **gekennzeichnet durch** eine Temperatursonde im Gasschlauch (7).

6. Vorrichtung gemäß mindestens einem der Ansprüche 1-5, **dadurch gekennzeichnet, dass** die Kunststoffkammer (2) ein bis fünf im Wesentlichen zylinderförmige Befeuchtungsmittel (8) enthält.

7. Vorrichtung gemäß 2. Anspruch 6, **dadurch gekennzeichnet, dass** die ein bis fünf im wesentlichen zylinderförmigen Befeuchtungsmittel (8) so im Gasstrom positioniert sind, das der Gasstrom über eine Zylinderstirnfläche (9) geführt wird oder
dass die ein bis fünf im wesentlichen zylinderförmigen Befeuchtungsmittel (8) so im Gasstrom positioniert sind, das der Gasstrom über die Zylindermantelfläche (10) geführt wird.

8. Vorrichtung gemäß mindestens einem der Ansprüche 6 oder 7, **dadurch gekennzeichnet, dass** die ein bis fünf im wesentlichen zylinderförmigen Befeuchtungsmittel (8) und/oder das erste Befeuchtungsmittel (3) beheizt werden.

9. Vorrichtung gemäß mindestens einem der Ansprüche 1-8, **dadurch gekennzeichnet, dass** der Gasschlauch (7) durch einen Heizdraht (11) beheizt wird.

## Claims

1. A device for gas humidifying in laparoscopy for connection to a gas supply device (insufflator), comprising a water reservoir (1) in a plastic chamber (2) with first absorbent humidifying means (3),
the plastic chamber having a gas inlet (4) and a gas outlet (5),
the gas inlet being connected to the insufflator directly or via an optional hose (6), and the gas outlet (5) leading to a gas hose (7),
one or more substantially cylindrical humidifying means (8) of a second absorbent material,
a gas hose (7), through which gas can be delivered to a patient,
the one or more substantially cylindrical humidifying means (8) of a second absorbent material having at least one cylinder front surface (9) being in contact with the water reservoir (1}, the other cylinder front surface or the cylinder shell (10) being positioned in the gas flow, **characterized by** that the water reservoir with the first humidifying means (3) is arranged concentrically around the gas flow.

2. The device according to claim 1, **characterized by** that the first and/or the second humidifying means (3, 8) consist of a material capable of transporting liquid by capillary action.

3. The device according to claim 1, **characterized by** that the plastic chamber (2) contains an access (12) for humidifying the one or more humidifying means (3, 8).

4. The device according to at least one of claims 1 to 3, **characterized by** a heated gas hose (7) at the gas outlet of the plastic chamber (2).

5. The device according to at least one of claims 1 to 4, **characterized by** a temperature sensor in the gas hose (7) .

6. The device according to at least one of claims 1 to 5, **characterized by** that the plastic chamber (2) contains one to five substantially cylindrical humidifying means (8) .

7. The device according to claim 6, **characterized by** that the one to five substantially cylindrical humidifying means (8) are positioned in the gas flow in such a way that the gas flow is guided over a cylinder front face (9) or
that the one to five substantially cylindrical humidifying means (8) are positioned in the gas flow in such a way that the gas flow is guided over the cylinder shell (10).

8. The device according to at least one of claims 6 or 7, **characterized by** that the one to five substantially cylindrical humidifying means (8) and/or the first humidifying means (3) are heated.

9. The device according to at least one of claims 1 to 8, **characterized by** that the gas hose (7) is heated by means of a heating wire (11).

## Revendications

1. Dispositif d'humidification de gaz en laparoscopie pour le raccordement à un dispositif d'alimentation en gaz (insufflateur), contenant un réservoir d'eau (1) dans une chambre en matière plastique (2) avec un premier moyen d'humidification absorbant (3),
la chambre en plastique présentant une entrée de gaz (4) et une sortie de gaz (5),
l'entrée de gaz étant reliée directement ou par l'intermédiaire d'un tuyau (6) optionnel à l'insufflateur et la sortie de gaz (5) menant à un tuyau de gaz (7),
un ou plusieurs moyens d'humidification (8) sensiblement cylindriques en un deuxième matériau absorbant,
un tuyau de gaz (7) par lequel du gaz peut être amené à un patient,
l'un ou les plusieurs moyens d'humidification (8) sensiblement cylindriques en un deuxième matériau absorbant étant en contact avec le réservoir d'eau (1) par au moins une surface frontale cylindrique (9), l'autre surface frontale cylindrique ou l'enveloppe cylindrique (10) étant positionnée dans le flux de gaz, et
**caractérisé en ce que** le réservoir d'eau avec le premier moyen d'humidification. (3) est disposé de manière concentrique autour du flux de gaz.

2. Dispositif selon la revendication 1, **caractérisé en ce que** le premier et/ou le deuxième moyen d'humidification (3, 8) est constitué d'un matériau capable de transporter du liquide par capillarité.

3. Dispositif selon la revendication 1, **caractérisé en ce que** la chambre en matière plastique (2) comporte un accès (12) pour humidifier le moyen ou les moyens d'humidification (3, 8) .

4. Dispositif selon au moins une des revendications 1 à 3, **caractérisé par** un tuyau de gaz chauffé (7) à la sortie de gaz de la chambre en matière plastique (2).

5. Dispositif selon au moins une des revendications 1 à 4, **caractérisé par** une sonde de température dans le tuyau de gaz (7).

6. Dispositif selon au moins une des revendications 1 à 5, **caractérisé en ce que** la chambre en matière plastique (2) contient de un à cinq moyens d'humidification (8) sensiblement cylindriques.

7. Dispositif selon la revendication 6, **caractérisé en ce que** les un à cinq moyens d'humidification (8) sensiblement cylindriques sont positionnés dans le flux de gaz de telle sorte que le flux de gaz soit guidé sur une surface frontale cylindrique (9) ou
**en ce que** les un à cinq moyens d'humidification (8) sensiblement cylindriques sont positionnés dans le flux de gaz de telle sorte que le flux de gaz soit guidé sur l'enveloppe cylindrique (10).

8. Dispositif selon au moins une des revendications 6 ou 7, **caractérisé en ce que** les un à cinq moyens d'humidification (8) sensiblement cylindriques et/ou le premier moyen d'humidification (3) sont chauffés.

9. Dispositif selon au moins une des revendications 1 à 8, **caractérisé en ce que** le tuyau de gaz (7) est chauffé par un fil chauffant (11).
